# EUROPEAN PATENT APPLICATION

(11) **EP 4 656 243 A2**
(43) Date of publication of application: **03.12.2025**
(21) Application number: 25200805.7
(22) Date of filing: 14.05.2018
(51) Int. Cl.: A61P 25/00

(54) **NFKF AND/OR NFKL FOR USE AS SYNTHESIS INTERMEDIATE FOR THE PREPARATION OF COMPOUNDS OF PHARMACEUTICAL INTEREST**

(30) Priority: 15.05.2017 BR 102017010169; 11.10.2017 WO PCT/BR2017/050314
(62) Divisional of application: 18802073.9
(71) Applicant: Remer Consultores Assessoria Empresarial Ltda., 20010-020 Rio De Janeiro (BR); Proteimax Biotecnologia Ltda, 05581001 São Paulo (BR)
(72) Inventor: Remer, Ricardo Amaral, Rio de Janeiro (BR); Heimann, Andrea Sterman, 04531-000 São Paulo (BR)
(74) Representative: Hübscher & Partner Patentanwälte GmbH

(57) **Abstract**

The present invention is in the fields of pharmaceutical sciences, chemistry and biotechnology. More specifically, the present invention describes a synthetic intermediate for the preparation of compounds of pharmaceutical interest. The synthesis intermediate of the present invention is peptidic and showed surprising stability and ease of handling when compared to the more closely related peptidic compounds.

## Description

### Field of the Invention

The present invention is in the fields of pharmaceutical sciences, chemistry and biotechnology. More specifically, the present invention describes a synthetic intermediate for the preparation of compounds of pharmaceutical interest. The synthetic intermediate of the present invention is peptidic and showed surprising stability and ease of handling when compared to the more closely related peptidic compounds.

### Background of the Invention

The peptidic compound which is a synthetic intermediate of the invention is surprisingly stable at extreme temperatures, does not pose the problem of fibril formation and provides for ease of handling in the preparation of products of pharmaceutical interest, including ligands for diagnostics and pharmaceutical compositions.

The peptidic compound which is closest to the present invention is hemopressin and its larger size variants with biological activity. However, hemopressin presents technical difficulties such as instability and tendency to form fibrils, which limit its use in pharmaceutical preparations (Bomar MG & Galande AK, Modulation of the cannabinoid receptors by hemopressin peptides. 520-524). These limitations have diminished initial enthusiasm with hemopressin: its natural tendency for aggregation/fibril formation substantially limits the range of possible concentrations at both the synthesis, pharmaceutical preparation and therapeutic use. The same literature (Bomar & Galande) goes so far as to speculate that hemopressin self-assembly may be physiologically relevant and potentially pathogenic or neurotoxic, analogously to the amyloid peptidic fibrils associated with Alzheimer's disease, Parkinson's disease and type II diabetes.

The results of the present invention are surprising in view of the closest background, and the present inventors are not aware of any report or suggestion in the literature that the peptidic compound of the invention would be more stable and more easily manipulable than Hemopressin, thus being in relation to this, an unexpected improvement and of unexpected magnitude.

The cannabinoid system, which comprises the CB1 and CB2 receptors and their endogenous ligands, acts on various metabolic functions, including control of food intake, energy and/or lipid metabolism, regulation of intestinal motility, immune system, in the balance of the calcium cycle, among others. Cannabinoid receptors are widely expressed in the brain, including cortex, hippocampus, amygdala, pituitary, and hypothalamus. CB receptors, particularly CB1, have already been identified in numerous peripheral organs and tissues, including the thyroid gland, adrenal gland, reproductive organs, adipose tissue, liver, muscle, and gastrointestinal tract.

It should also be noted that studies with peptides resembling hemopressin (pepcans) have been facing several challenges. For example, manipulation of said larger peptides has been hindered by fibril aggregation/formation.

Background searches have disclosed documents only partially relevant for the present invention. Such documents will be described below, being set forth herein solely for the purpose of serving as a basis for the state of the art, since none of them anticipates or even suggests the object of the present invention.

Some studies presented by one of the present inventors in *Novel Peptide Substrates for Endopeptidase 24.15 Neurolysin and Angiotensin Converting Enzyme* (Vanessa Rioli, Fabio C. Gozzo, Andrea S. Heimann, Alessandra Linardi, Jose E. Krieger, Claudio S. Shida, Paulo C. Almeida, Stephen Hyslop, Marcos N. Eberlin, Emer S. Ferro; March 7, 2003) demonstrate an effective technique of "screening" new peptides. The present invention differs from that document, among other reasons, because it provides a different molecular entity. It also provides a more stable compound.

Furthermore, Gomes et al. disclose the natural presence of certain endocannabinoids in the brain of mice (ie, local expression) having found N-extended versions of Hp. In addition, one of the conclusions of Gomes et al is that the N-extended versions found in the brain (RVD-Hpα and VD-Hpα) have *opposite* function to that of the peptide of the present invention, this being antagonist/inverse agonist and those being agonist of cannabinoid receptors. Consequently, not only Gomes et al do not disclose the use of the peptide of the invention as synthetic intermediate, but the person skilled in the art, from reading Gomes et al., would not be motivated to: (i) synthesize the peptidic compound of the invention; (ii) expect its stability to be substantially greater than that of Hemopressin; (iii) test it for the other uses indicated herein with reasonable expectation of success. One skilled in the art would not expect said peptidic compound of the invention to have greater stability.

Document WO 2011/011847 (=US2015/297669, US9,452,196, EP2459207) has as one of the inventors the inventor of the present invention (Heimann). This document only discloses the use of hemopressin (Hp) for the control of diabetes and obesity/weight gain and is limited to Hp and larger derivatives such as DV-Hp, PVD-Hp, RVD-Hp. The present invention differs from said document, among other reasons, for disclosing a different compound, which provides benefits when compared to hemopressin, including more ease of preparation, greater stability.

Other references of scientific literature which circumscribe the invention, but without anticipating or suggesting it, include the following documents.

ASPRONI, B. et al. Novel pyrrolocycloalkylpyrazole analogues as CB1 ligands. Chemical Biology and Drug Design, p. 1-13, 2017.

HILDEBRANDT, A. K. et al. Efficient computation of root mean square deviations under rigid transformations. Journal of Computational Chemistry, v. 35, p. 765-771, 2014.

HUA T. et al. Crystal structure of the human cannabinoid receptor CB1. Cell, v. 167, p. 750-762, 2016.

MAIOROV, V. N.; CRIPPEN, G. M. Significance of root-mean-square deviation in comparing three-dimensional structures of globular proteins. Journal of Molecular Biology, v. 235, p. 625-634, 1994.

MORGAN, C. A.; HURLEY, T. D. Characterization of two distinct structural classes of selective aldehyde dehydrogenase 1A1 inhibitors. Journal of Medicinal Chemistry, v. 58, p. 1964-1975, 2015.

PERTWEE, R. G. The diverse CB1 and CB2 receptor pharmacology of three plant cannabinoids: Δ9-tetrahydrocannabinol, cannabidiol and Δ9 -tetrahydrocannabivarin. British Journal of Pharmacology, v. 153, p. 199-215, 2008.

RAMACHANDRAN, G. N.; RAMAKRISHNAN, C.; SASISEKHARAN, V. Stereochemistry of polypeptide chain configurations. Journal of Molecular Biology, v. 7, p. 95-99, 1963.

SANTOS, P. S. et al. Efeitos do ácido lipóico nas concentrações de glutamato e taurina no hipocampo de ratos após convulsões induzidas por pilocarpina. Arq. Neuro-Psiquiatr. [online]. 2011, vol.69, n.2b, pp.360-364.

MUNRO et al. Nature 365:61-65, 1993.

RINALDI-CARMONA M. et al. J. Pharmacol. Exp. Ther. 278:871-878, 1996.

Gupta A, Heimann AS, Gomes I, Devi LA. Antibodies against G-protein coupled receptors: novel uses in screening and drug development. Comb. Chem High Throughput Screen. 2008, 11(6)463-467.

Langmead CJ1, Watson J, Reavill C. Muscarinic acetylcholine receptors as CNS drug targets. Pharmacol Ther. 2008 Feb;117(2):232-43. Epub 2007 Dec 20.

Bomar MG & Galande AK. Modulation of the cannabinoid receptors by hemopressin peptides. Life Sci. 2013 92(8-9): 520-524.

Dvorácskó S, Tömböly C, Berkecz R, Keresztes A. Investigation of receptor binding and functional characteristics of hemopressin(1-7). Neuropeptides 2016, 58:15-22.

Gomes I, Grushko JS, Golebiewska U, Hoogendoorn S, Gupta A, Heimann AS. Ferro ES, Scarlata S, Fricker LD, and Devi LA. Novel endogenous peptide agonists of cannabinoid receptors. FASEB J. 2009 Sep; 23(9): 3020-3029.

Gelman JS, Sironi J, Castro LM, Ferro, ES, and Fricker LD. Hemopres-sins and other hemoglobin-derived peptides in mouse brain: Comparison between brain, blood, and heart peptidome and regulation in Cpefat/fat mice. J Neurochem. 2010 May; 113(4): 871-880.

Bauer M, Chicca A, Tamborrini M, Eisen D, Lerner R, Lutz B, Poetz O, Pluschke G, Gertsch J. Identification and Quantification of a New Family of Peptide Endocannabinoids (Pepcans) Showing Negative Allosteric Modulation at CB 1 Receptors. The Journal of Biological Chemistry Vol. 287, No. 44, pp. 36944-36967, October 26, 2012.

Reichwein JF & Liskamp RMJ. Site-specific N-alkylation of peptides on the solid phase. Tetrahedron Letters, Volume 39, Issue 10, 5 March 1998, Pages 1243-1246.

Szlavicz E, Perera PS, Tomboly C, Helyes Z, Zador F, Benyhe S, Borsodi A, Bojnik E. Further Characterization of Hemopressin Peptide Fragments in the Opioid and Cannabinoid Systems. Anesth Analg. 2015 Dec;121(6):1488-94. doi: 10.1213/ANE.0000000000000964. PubMed PMID: 26465932.

Special Periodic Reports, Amino Acids, Peptides and Proteins: Volume 42, Royal Society of Chemistry, 2013.

From the reviewed literature, no document was found anticipating or suggesting the teachings of the present invention, let alone their surprising technical effects, so that the solution proposed here, in the eyes of the inventors, has novelty and inventive step over the state of the art.

### Summary of the Invention

The present invention addresses a number of known problems of the prior art, for example, to provide: a peptide compound which is more stable and easier to handle than hemopressin and useful as synthetic intermediate for the preparation of compounds of pharmaceutical interest; a molecular entity which provides these and/or other technical effects without the disadvantages arising from the use of hemopressin such as instability and formation of aggregates.

It is an object of the invention to provide a synthetic intermediate in the preparation of compounds of pharmaceutical and/or diagnostic interest which comprise the peptidic compound and may include a chemical modification which is a cyclization and/or an inclusion of other functional group which is biotin, amide, alkyl, alcoxy, halogen, hydroxy, or PEG group, a non-natural aminoacid, a D-aminoacid, its salts; and/or combinations thereof.

The synthesis intermediate of the invention is a peptidic compound of formula: NFKF, NFKL, its salts, or combinations thereof.

The compound of the invention is synthetic and distinct from known natural forms and is useful for preparing a product of pharmaceutical interest selected from a ligand for diagnostic use and a curative or prophylactic medicament for a mammal.

This object of the present patent application will be immediately appreciated by those skilled in the art and by companies having interests in this segment, and will be described in sufficient detail for its reproduction in the following description.

### Brief Description of the Figures

The following detailed description and the accompanying figures illustrate the main features and embodiments of the present invention, set forth in greater detail to provide further support to the person skilled in the art, and so that she/he may understand and reproduce the inventive concept of the invention in any of its embodiments. Such details or figures are not to be construed as limiting and serve only to illustrate some of the embodiments of the present invention.

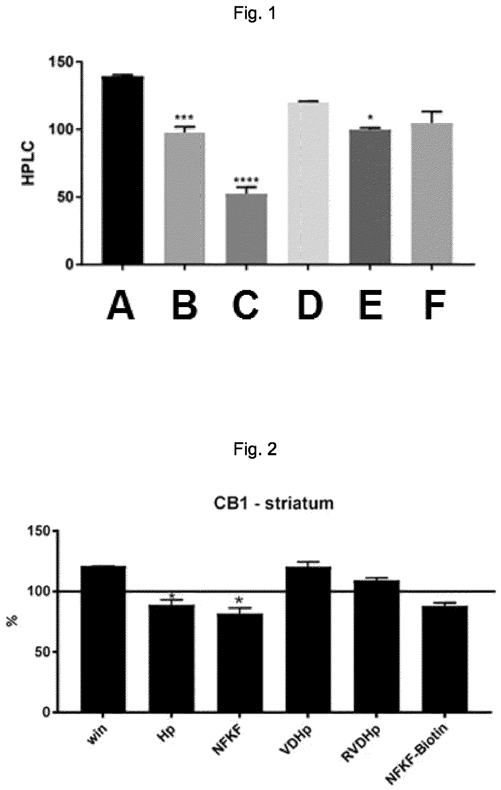

Figure 1 shows the results of comparative stability tests of the compound of the invention in the NFKF embodiment vs. Hp (PVNFKFLSH). The data on the graph shows the results of the concentration measurement of each of these actives by HPLC, after freezing for 24 hours or separately after heating at 100°C for 10 minutes (n = 2). In the vertical axis the area of the HPLC peak is shown. In A) is shown the Hp (control); in B) Hp after freezing for 24 hours; in C) Hp heated to 100°C; in D) the NFKF (control); in E) NFKF after freezing for 24 hours; in F) NFKF heated to 100°C. Statistical significance data are also shown, the asterisks indicating: (*) P <0.05 vs Control (***) P <0.005 vs Control; (****) P <0.0001 vs Control.
Figure 2 shows results of the binding assays of various compounds to the CB1 receptor in anti-CB1 antibodies sensitive to activation/conformational changes of receptor. Treatment with 1 µM. The values in % of binding to the CB1 receptor in relation to control are indicated. * P <0.05 vs control.

### Detailed Description of the Invention

The inventive concept underlying the object of the invention is a synthesis intermediate of formula: NFKF, NFKL, its salts, or combinations thereof.

The synthesis intermediate of the invention is synthetic and distinct from known natural forms and is useful for preparing a product of pharmaceutical interest selected from a ligand for diagnostic use and a curative or prophylactic medicament for a mammal.

The compound of the invention is a synthetic intermediate in the preparation of compounds of pharmaceutical interest which comprise the peptidic compound of the invention and include a chemical modification and/or another functional group.

For purposes of the present invention the following definitions are used.

### Product of pharmaceutical interest

In the context of the present application, "compound of pharmaceutical interest" means any molecular entity comprising the compound described as inventive concept of the present application, to obtain molecular entities obtained by chemical modification/derivatization of the same, or with the inclusion of other functional groups, linear or branched side chains, alteration of hydrophilicity or hydrophobicity, among others, provided that they comprise as nucleus the entity NFKF or NFKL as defined above, except for the natural and already known entities.

### Modified peptide

In the context of the present application, "modified peptide" is to be understood as a non-naturally occurring, artificially modified or synthesized peptide, including halides, cyclized, amidated, alkylated, alkoxylated, hydroxylated, PEGylated forms, forms with other functional groups on any amino acid, or salt forms thereof, as well as on an amino acid or peptide, including the non-natural, such as d-amino acid forms. The peptidic compound may be pegylated using techniques known to those skilled in the art, such as, for example, pegylation with reagents containing the succinimidyl group, which preferentially react with primary amines present in the N-terminal region of the peptide. The synthesis intermediate of the invention may be alkylated at any amino acid using techniques known to those skilled in the art, including, for example, the Mitsunobu reaction described in Reichwein & Liskamp (Reichwein JF & Liskamp RMJ, Site-specific N-alkylation of peptides on the solid phase, Tetrahedron Letters, Volume 39, Issue 10, 5 March 1998, Pages 1243-1246). Said article describes the introduction of any alkyl group into a specific amide function of a peptide. The synthesis intermediate of the invention may be alkoxylated, substituted with halogens, hydroxy or other functional groups on any amino acid using techniques known to those skilled in the art, including, for example, those described in the book Special Periodic Reports, Amino Acids, Peptides and Proteins: Volume 42, Royal Society of Chemistry, 2013. The peptidic compound of the invention may be modified with other molecular species useful in diagnostic and/or therapeutic applications, such as Biotin, using techniques known to those skilled in the art.

### Cyclic or circular peptide

In the context of the present application, "cyclic, cyclized or circular peptide" is to be understood as a peptide which has a covalent bond between the two ends of a linear peptide molecule by any method known in the art, particularly by the activity of enzymes. The cyclic peptide can be used instead of the linear peptide because it is more difficult to degrade, since its ends or zones of attack by hydrolyzing enzymes are not as exposed as in a linear peptide.

### Ligand of CB receptor

In the context of the present patent application, a "ligand of CB receptor" is to be understood as a compound or molecule that interacts with the CB system and/or with CB1 or CB2 receptors.

### Modulating the CB receptor function or the cannabinoid system

In the context of the present patent application, it is meant to "modulate the CB receptor function" as an interaction that results in the alteration of the biochemical activity of the CB receptor, particularly CB1 or CB2. It is understood that the change is positive when an antagonist or inverse agonist effect occurs at CB receptors and that the change is negative when an agonist effect occurs at CB receptors. The results of the tests presented in the present patent application indicate that the compound of the invention interacts with and/or modulates the CB1 receptor and/or the CB2 receptor, probably as an allosteric modulator of CB1 and/or CB2 receptor. Thus the compound of the invention is useful for modulating the cannabinoid system, either by modulating the CB1 receptor, or the CB2 receptor, of both concomitantly, by modulating the binding or action of other substances interacting in the cannabinoid system, by modulating proteases or peptidases which lead to the generation or degradation of active peptides in the cannabinoid system, or by improving the natural system by the protection of natural molecules with the compounds of the invention, that is, the compound of the invention could also lead the natural system to a protective action independently of the binding to said receptors, or combinations of said effects.

### Modulating the function of muscarinic receptors

In the context of the present application, the term "modulating the function of muscarinic receptors " should be understood as an interaction which leads to alterations in muscarinic acetylcholine receptors (mAChRs), which play an important role in cognitive functions, such as learning and memory, control of dopamine release, modulation of locomotor activity, its modulation being also useful in the control of Alzheimer's disease and/or control of dependency or addiction to abuse drugs. It is understood that the change is positive when an antagonist or inverse agonist effect occurs at muscarinic receptors and that the change is negative when an agonist effect occurs at muscarinic receptors. The tests presented in the present patent application suggest that the compound of the invention interacts with and/or modulates muscarinic receptors.

The results of *in vitro* tests have shown that the synthesis intermediate of the invention provides advantages in the preparation of pharmaceutical compositions and in their stability.

The synthesis intermediate of the invention has proved to be much more stable than hemopressin, which in addition causes technical problems of fiber formation or fibrils, as well as its known variants.

The peptidic compound of the invention has also been shown to be an appropriate synthetic intermediate for the preparation of other compounds useful in diagnostic and/or therapeutic applications. Tests conducted in the present invention show that the synthesis intermediate of the invention is suitably modified with biotin known to those skilled in the art as useful in diagnostic preparations or kits. The synthesis intermediate compound of the invention, when modified/protected by biotin, has also been shown to bind to the CB1 receptor in tests with anti-CB1 antibodies sensitive to the activation/conformational changes of the receptor.

The following examples are only intended to exemplify some of the various ways of practicing the invention, however, without limiting the scope thereof.

Comparative tests showed that the synthesis intermediate of the invention showed superior *in vitro* stability at temperature extremes when compared to hemopressin.

### Examples

### Example 1. Stability tests under extreme conditions - Superiority of the synthesis intermediate of the invention in relation to Hemopressin

In this embodiment, the stability of the synthesis intermediate of the invention in the NFKF embodiment was compared to that of hemopressin (Hp, PVNFKFLSH) under extreme conditions. Hp is known to have the problem of fibril formation, as well as variants thereof which have a greater number of amino acids. Samples of NFKF and Hp were subjected to two separate tests, that of stability by freezing for 24 hours and heating at 100°C for 10 minutes.

The results of figure 1 show that, by freezing for 24h, a significant part of Hp is lost or degraded (from 140 to 97, i.e. approximately 31%), as evidenced by measurements by HPLC (analytical column of 2.1 mm, with gradient running of 10-60% B. Solvent A is water/0.1% TFA and solvent B is acetonitrile/0.075% TFA). The synthesis intermediate of the invention in the NFKF embodiment, on the other hand, remained much more stable and suffered substantially less degradation (from 120 to 100, ie, 16.7%). The results of Figure 1 also show that upon heating at 100°C for 10 minutes, even more significant part of the Hp is lost or degraded (from 140 to 50, ie approximately 64.3%), as evidenced by measurements by HPLC. On the other hand, the compound of the invention in the NFKF embodiment remained stable and did not suffer statistically significant degradation.

Together, these data show that the synthesis intermediate of the invention provides much greater stability and lower degradation under extreme temperature conditions, which favors it in the manipulation, galenics, pharmaceutical preparations and stability of the pharmaceutical both in the post-acquisition phase of the active principle, and in the industrial production of medicines and, not least, in the transport logistics chain.

### Example 2. Use of the compound NFKF as the synthesis intermediate in the preparation of other compounds

In the present application, the synthesis intermediate NFKF is used as a synthetic intermediate in obtaining other compounds of pharmaceutical/diagnostic interest. In this and other embodiments the compound is referred to as a "modified peptide", which is unnatural, being artificially modified or obtained by synthesis, including halides, cyclized, amidated, alkylated, alkoxylated, hydroxylated, PEGylated, other functional groups in any amino acids, or salt forms thereof, as well as modified with an amino acid or peptide, including the non-natural as the d-amino acid forms. In the embodiment of this example, the peptidic compound of the invention has been modified with molecular species useful in diagnostic and/or therapeutic applications, such as Biotin, using techniques known to those skilled in the art. Biotin is a label or tag that can be used in the detection of the ligand target of the invention, in the present case the cannabinoid and/or muscarinic receptors, through the use of anti-biotin antibodies, or avidin/streptavidin detection strategies with enzymes such as horseradish peroxidase, alkaline phosphatase, or fluorescent probes.

The synthesis intermediate of the invention modified with biotin in an embodiment was used in screening techniques using anti-CB1 receptor antibodies sensitive to conformational changes.

Antibodies sensitive to CB1 receptor activation were generated and characterized by their specificities. For screening compounds, Striatum membranes (5µg per well) were plated on NUNC-Immuno 96-well plates (Nalge-Nunc) and dried at room temperature. Membranes were washed with PBS (phosphate buffered saline) and incubated with or without different ligands (1µM) and the extent of receptor recognition by the antibodies was measured by ELISA. The results of the tests are shown in Table 1, which indicates the% binding to the CB1 receptor in relation to control:

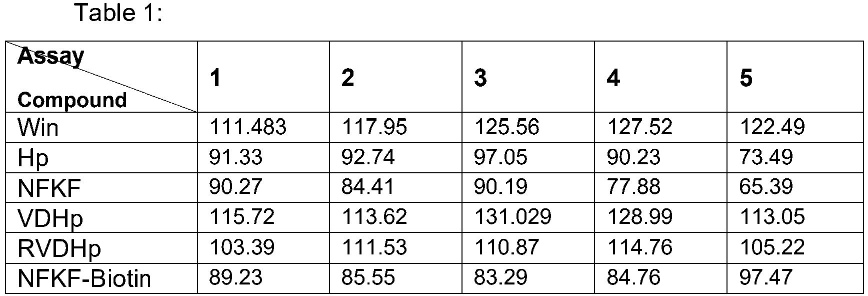

Figure 2 shows these results and confirms that the synthetic intermediate of the invention has been shown to be an appropriate synthetic intermediate for the preparation of other compounds useful in diagnostic and/or therapeutic applications. The synthetic intermediate of the invention, when modified/protected by biotin, has also been shown to bind to the CB1 receptor in tests with anti-CB1 antibodies sensitive to the activation/conformational changes of the receptor.

In this context, the present co-inventor has already shown (Gupta et al) that µ and δ opioid receptors dimerize with CB1 cannabinoid receptors and that heterodimerization leads to modulation of receptor function. In addition, the use of other CB1 receptor binding substances provides increased recognition of µ opioid receptors, apparently mediated by conformational changes in them. These observations support the idea that conformational changes caused by CB receptor ligands may affect the receptor partner in a heterodimer and that this can be detected using antibodies sensitive to conformational changes. Thus, the test results in this example support the concept of an *in vitro* diagnostic process for protein-protein or peptide-protein interactions that modulate the function of GPCRs (G protein coupled receptors, or G-Protein Coupled Receptors/GPCRs), including heterodimeric interactions.

## Claims

1. Synthesis intermediate for the preparation of compounds of pharmaceutical and/or diagnostic interest **characterized by** formula: NFKF, NFKL, its salts, or combinations thereof.

2. Synthesis intermediate according to claim 1 wherein said preparation includes cyclization of NFKF, NFKL, its salts, or combinations thereof and/or inclusion of a functional group which is biotin, amide, alkyl, alcoxy, halogen, hydroxy, or PEG group, a non-natural aminoacid, a D-aminoacid, its salts; and/or combinations thereof.

3. Synthesis intermediate according to claim 1 or 2 for use in the preparation of NFKF-biotin.

4. Compound of formula NFKF, NFKL, its salts, or combinations thereof for use as a synthesis intermediate for the preparation of compounds for modulating the CB receptor function or the cannabinoid system.

5. Compound according to claim 4 comprising (i) NFKF, NFKL, its salts, or combinations thereof, and (ii) a functional group which is biotin, amide, alkyl, alcoxy, halogen, hydroxy, or PEG group, a non-natural aminoacid, a D-aminoacid, its salts; and/or combinations thereof.
